# EUROPEAN PATENT APPLICATION

(11) **EP 1 693 059 A1**
(43) Date of publication of application: **23.08.2006**
(21) Application number: 05003829.8
(22) Date of filing: 22.02.2005
(51) Int. Cl.: A61K 31/343, A61P 29/00

(54) **Use of rocaglamide derivatives as NF-AT-specific inhibitors for the treatment of certain inflammatory diseases**

(71) Applicant: Deutsches Krebsforschungszentrum, 69120 Heidelberg (DE)
(72) Inventor: Li-Weber, Min, 67098 Bad Dürkheim (DE); Krammer, Peter H., 69120 Heidelberg (DE); Proksch, Peter, 52428 Jülich (DE)
(74) Representative: Huhn, Michael

(57) **Abstract**

Disclosed is the use of a rocaglarnide derivative of the formula (I) for the manufacture of a medicament which contains a rocaglamide derivative of formula (I) for the treatment of NF-AT-dependent inflammatory diseases, pharmaceutical compositions comprising rocaglamide derivatives and methods of producing such pharmaceutical compositions.

## Description

The present invention relates to the treatment of inflammatory diseases by specifically inhibiting NF-AT-dependent transcriptional activity.

Inflammatory diseases arise from inappropriate activation of the immune system leading to abnormal expression of genes encoding inflammatory cytokines and tissue-destructive enzymes. During an immune response T helper (Th) cells produce various cytokines required for an efficient suppression of infections. Th type 1 (Th1) cytokines Interferon-γ (IFN-γ) and tumor necrosis factor-α (TNF-α) promote cell-mediated immunity and Th2 cytokines interleukin-4 (IL-4), IL-5, IL-6, IL-10 and IL-13 promote humoral (antibody) immunity. However, uncontrolled expression of these cytokines is dangerous and causes inflammatory diseases, including rheumatoid arthritis, diabetes and hepatitis or to atopic disorders including allergen-induced asthma, rhinoconjunctivitis and anaphylaxis. Many inflammatory genes, e.g. those coding for IFN-γ, TNF-α and IL-4, are regulated at the transcriptional level by pro-inflammatory transcription factors such as NF-kappa B, AP-1 (Fos/Jun) and NF-AT. In resting T cells, NF-kappa B is sequestered into an inactive state by the cytoplasmic inhibitor of NF-kappa B (I-kappa B). T cell activation leads to the rapid activation of the I-kappa B kinases (IKKs), and results in phosphorylation, ubiquitylation and subsequent degradation of I-kappa B proteins, which allows nuclear translocation of NF-kappa B, In contrast, NF-AT family proteins are calcium and calcineurin-regulated transcription factors. In resting T cells, NF-AT proteins are phosphorylated and reside in the cytoplasm. T cell activation leads to activation of the Ca²⁺-dependent phosphatase calcineurin resulting in rapid dephosphorylation of NF-AT and its translocation to the nucleus.

From the prior art it is known that inflammatory skin diseases and allergic inflammatory disorders such as asthma can be successfully treated with extracts of *Aglaia* plants (family Meliaceae). It has been demonstrated that the active compounds of these plants, so-called rocaglamide, respectively cyclopentabenzofuran derivatives, possess antiproliferative (see e.g. US 4,539,414; Bohnenstengel et al., 1999, Z. Naturforsch [C]. Vol. 54, pages 55-60) and insecticidal activity (Schneider et al., 2000 Phytochemistry. Vol. 54, pages 731-6). Furthermore, the patent application DE 198 35 325 discloses the use of cyclopentabenzofuran derivatives for the treatment of diseases related to the pro-inflammatory transcription factor NF-kappa B (see the description SUPRA), giving a hint to a possible mode of action for the anti-inflammatory activity of rocaglamide derivatives, namely by inhibition of NF-kappa B (see also Baumann et al., 2002, J Biol Chem., Vol. 277, pages 44791-800). Consistently, the DE 101 58 561 discloses the use of cyclobenzofuran derivatives for the treatment of Parkinson's Disease, as it is known from the prior art that in patients suffering from Parkinson's Disease an increased activity of NF-kappa B-mediated signal transduction is observed.

However, an inhibition of NF-kappa B which is achieved with the rocaglamide doses disclosed in the prior art bears the disadvantage that it may lead to an undesired programmed cell death (apoptosis), since it is known to the person skilled in the art that NF-kappa B exhibits anti-apoptotic properties (see Voll et al., Immunity 2000, Vol. 13(5), pages 677-89; Pikarsky et al., Nature 2004, Aug. 25, [electronically published ahead of print]). Consequently, the problem underlying the present invention resides in providing compounds useful for treating inflammatory and/or other diseases while circumventing an inhibition of NF-kappa B and/or AP-1 and circumventing an induction of undesired apoptosis.

The inventors have now surprisingly found, that rocaglamides are potent immunosuppressive phytochemicals that suppress IFN-γ, TNF-α, IL-2 and IL-4 production in peripheral blood T cells at nM concentrations. It has also been found that rocaglamides, at the doses that inhibit cytokine production, selectively inhibit activities of NF-AT without impairing the NF-kappa B and AP-1 activities. Consequently, rocaglamides may serve as a new source of NF-AT-specific inhibitors for treatment of certain inflammatory diseases.

The problem of the present invention is therefore solved by the use of a rocaglamide derivative of the formula (I) 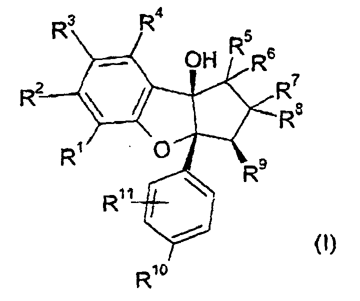 wherein
R¹ is selected from hydrogen, halogen and alkyl;
R² is selected from halogen, alkyl and alkoxy;
R³ is selected from hydrogen, halogen and alkyl;
R⁴ is selected from halogen, alkyl and alkoxy;
   or R² and R³ together form a -OCH₂CH₂O- unit;
R⁵ is selected from hydroxy, acyloxy, amino, monoalkylamino, dialkylamino and -NR¹²-CHR¹³-COOR¹⁴, with
R¹² being selected from hydrogen and alkyl,
R¹³ being selected from phenyl and benzyl, which both may carry a substituent from the group hydroxy, indolyl and imidazolylmethyl, and alkyl which may be substituted by a group selected from OH, SH, alkoxy, thioalkoxy, amino, monoalkylamino, dialkylamino, carboxy, carboxyalkyl, carboxamide and guanidino groups;
   or R¹² and R¹³ together form a -(CH₂)₃- or -(CH₂)₄- group;
R¹⁴ being selected from alkyl and benzyl;
   in which case R⁶ is hydrogen; or
R⁵ and R⁶ together form an oxo or hydroxyimino group;
R⁷ is hydrogen;
R⁸ is selected from hydrogen, -COOR¹⁵ and CONR¹⁶R¹⁷, wherein
R¹⁵ and R¹⁶ are independently selected from hydrogen and methyl, and
R¹⁷ is selected from hydrogen, methyl, 4-hydroxybutyl and 2-tetrahydrofuryl;
R⁹ is selected from phenyl which is optionally substituted and hetaryl which is optionally substituted;
R¹⁰ is selected from hydrogen, halogen, alkyl and alkoxy, and
R¹¹ is selected from hydrogen, hydroxy, halogen, alkoxy and alkyl; or
   R¹⁰ and R¹¹ are in ortho-position to each other and together form a -OCH₂O- unit;
   or a pharmaceutically acceptable salt thereof, for the manufacture of a medicament for the treatment of inflammatory diseases,
wherein the rocaglamide derivative of the formula (I) is used in a therapeutically effective amount which enhances the phosphorylation of NF-AT in leukocytes of a patient but which does essentially not inhibit NF-kappa B activity and AP-I activity.

The term "alkyl", as mentioned in the above definitions of the substituents R¹ to R¹⁷, in each case refers to a substituted or an unsubstituted alkyl group, preferably an unsubstituted alkyl group. Furthermore, the term "alkyl", as mentioned in the above definitions of the substituents R¹ to R¹⁷, in each case preferably refers to a C₁- to C₄-alkyl group, namely methyl, ethyl, i-prapyl, n-propyl, n-butyl, i-butyl, sec-butyl or tert-butyl. The above also applies when "alkyl" is used in "alkylamino" and "dialkylamino" and other terms containing the term "alkyl".

The term "alkoxy", as mentioned in the above definitions of the substituents R¹ to R¹⁷, in each case refers to a substituted or an unsubstituted alkoxy group, preferably an unsubstituted alkoxy group. Furthermore, the term "alkoxy", as mentioned in the above definitions of the substituents R¹ to R¹⁷, in each case preferably refers to a C₁- to C₄₋alkoxy group, namely methoxy, ethoxy, i-propyloxy, n-propyloxy, n-butyloxy, i-butyloxy, sec-butyloxy or tert-butyloxy. The above also applies when "alkoxy" is used in "thioalkoxy" and other terms containing the term "alkoxy".

The term "acyloxy", as mentioned in the above definitions of the substituents R¹ to R¹⁷, in each case refers to a substituted or an unsubstituted acyloxy group, preferably an unsubstituted acyloxy group. Furthermore, the term "acyloxy", as mentioned in the above definitions of the substituents R¹ to R¹⁷, in each case preferably refers to a C₁- to C₄₋acyloxy group, namely formyloxy, acetoxy, i-propyloxy, n-propyloxy, n-butyloxy, i-butyloxy, sec-butyloxy or tert-butyloxy.

The term "hetaryl" as used in the above definition refers to a 5-,6- or 7-membered carbocyclic saturated or non-saturated, aromatic or non-aromatic ring which may carry in the ring one or more heteroatoms from the group O, S, P, N.

In a preferred embodiment of the present invention, the substituents R¹ and R¹⁴ have the following meanings:
R¹ and R³ each are hydrogen;
R² and R⁴ each are independently selected from methoxy which is optionally substituted;
R⁵ is selected from hydroxy, formyloxy and acetyloxy, alkylamino, -NR¹²-CHR¹³-COOR¹⁴, with
R¹² being selected from hydrogen and alkyl,
R¹³ being selected from: alkyl which may be substituted by: a group selected from OH, SH, alkoxy; thioalkoxy, amino, monoalkylamino, dialkylamino, carboxy, carboxyalkyl, carboxamide and guanidino groups; and phenyl and benzyl, which both may carry a substituent from the group hydroxy, indolyl and imidazolylmethyl;
R¹⁴ being selected from alkyl and benzyl;
R⁶ is hydrogen;
R⁷ is hydrogen:
R⁸ is selected from hydrogen, -COOCH₃ and CON(CH₃)₂ ;
R⁹ is phenyl which is optionally substituted;
R¹⁰ is methoxy,
R¹¹ is selected from hydrogen and hydroxy, or
R¹⁰ and R¹¹ are in ortho-position to each other and together form a -OCH₂O- unit.

In a more preferred embodiment of the present invention, the rocaglamide derivatives of the present invention refer to those of formula (I) wherein
R¹ and R³ each are hydrogen,
R² and R⁴ each are optionally substituted methoxy,
R⁵ is hydroxy or -NR¹²-CHR¹³-COOR¹⁴, with
R¹² being selected from hydrogen and alkyl,
R¹³ being selected from- alkyl which may be substituted by: a group selected from OH, SH, alkoxy; thioalkoxy, amino, monoalkylamino, dialkylamino, carboxy, carboxyalkyl, carboxamide and guanidino groups; and phenyl and benzyl, which both may carry a substituent from the group hydroxy, indolyl and imidazolylmethyl;
R¹⁴ being selected from alkyl and benzyl;
R⁶ and R⁷ each are hydrogen.
R⁸ is -CON(CH₃)₂,
R^{9 is} optionally substituted phenyl,
R¹⁰ is methoxy and
R¹¹ is hydrogen; or wherein
R¹ and R³ each are hydrogen,
R² and R⁴ each optionally substituted methoxy,
R⁵ is acetoxyor -NR¹²-CHR¹³-COOR¹⁴, with
R¹² being selected from hydrogen and alkyl,
R¹³ being selected from: alkyl which may be substituted by: a group selected from OH, SH, alkoxy; thioalkoxy, amino, monoalkylamino, dialkylamino, carboxy, carboayalkyl, carboxamide and guanidino groups; and phenyl and benzyl, which both may carry a substituent from the group hydroxy, indolyl and imidazolylmethyl;
R¹⁴ being selected from alkyl and benzyl;
R⁶ and R⁷ each are hydrogen,
R⁸ is -CON(CH₃)₂,
R⁹ is optionally substituted phenyl,
R¹⁰ is methoxy and
R¹¹ is hydrogen; or wherein

R¹ and R³ each are hydrogen,
R² and R⁴ each optionally substituted methoxy,
R⁵ is formyloxy or -NR¹²-CHR¹³-COOR¹⁴, with
R¹² being selected from hydrogen and alkyl,
R¹³ being selected from: alkyl which may be substituted by: a group selected from OH, SH, alkoxy; thioalkoxy, amino, monoalkylamino, dialkylamino, carboxy, carboxyalkyl, carboxamide and guanidino groups; and phenyl and benzyl, which both may carry a substituent from the group hydroxy, indolyl and imidazolylmethyl;
R¹⁴ being selected from alkyl and benzyl;
R⁶ and R⁷ each are hydrogen,
R⁸ is hydrogen or -COOCH₃,
R⁹ is optionally substituted phenyl, and
R¹⁰ and R¹¹ are in ortho-position to each other and together form a -OCH₂O- unit.

In a further embodiment of the present invention, R⁸ is a group of the formula

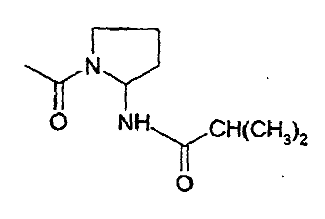

In still a further embodiment of the present invention, R⁵ and R⁸ together form a group of the formulae 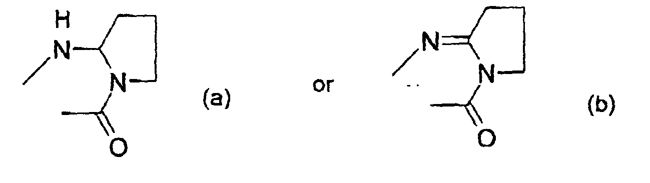 wherein
R⁵ is connected to the nitrogen.

As used herein, the term "rocaglamide derivative(s)" refers to cyclopenta[b]tetrahydrobenzofuran derivatives having the general formula (I). Examples of such compounds include compounds such as rocaglamide, aglaroxin C, cycloracaglamide, rocagtaol, methylrocaglate (aglafolin), desmethylrocaglamide, pannellin and the recently isolated dioxanyloxy-modified derivatives silvestrol and episilvestrol (Hwang et al., 2004, J. Org. Chem. Vol. 69: pages 3350-3358). Most preferably, the rocaglamide derivatives contemplated for the purposes of the present invention are those of formula (II) (named Roc-1 in the example section), formula (III) (named Roc-2 in the example section) and formula (IV) (named Roc-3 in the example section).

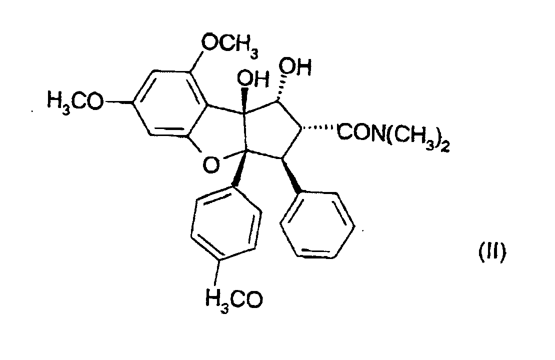

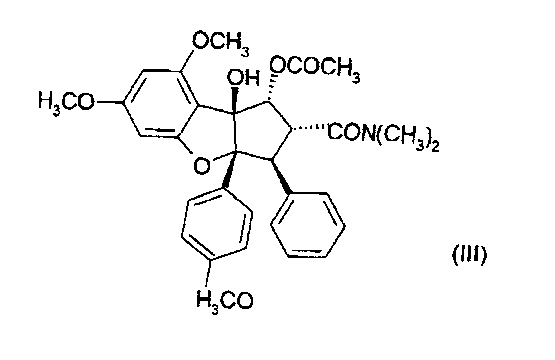

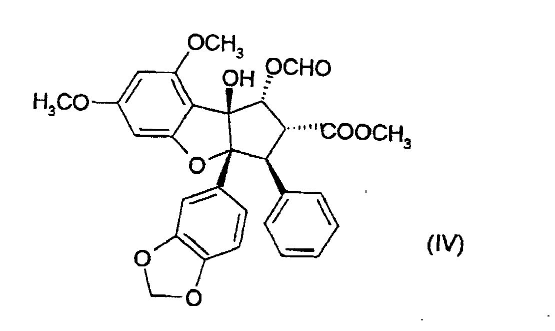

For the preparation of the rocaglamide derivatives according to the present invention, reference is made to WO 00/07579, WO 03/045375 and WO 00/08007.

Some of the compounds of the invention and/or salts or esters thereof will exist in different stereoisomeric forms. All of these forms are included in the present invention.

As used herein, the term "pharmaceutically acceptable salt" refers to those salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describe phannaceutically acceptable salts in detail in J. Pharmaceutical Sciences, 66: 1-19 (1977), which is incorporated herein by reference. The salts can be prepared in situ during the final isolation and purification of the rocaglamide derivatives, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, berate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydmxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate. 3-phenylpropionate, phosphate, picrate, pivalate, propionate, stearate, succinate, sulfate, tartrate, thiocyanate, p-tolueneswfonate, undecanoate, vale.rate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "treatment" as used in relation to the treatment of inflammatory diseases is to be understood as embracing both symptomatic and prophylactic modes, that is the immediate treatment, e.g. of acute inflammation (symptomatic treatment) as well as advance treatment to prevent, ameliorate or restrict long term symptomatology (prophylactic treatment). The term "treatment" as used in the present specification and claims in relation to such diseases is to be interpreted accordingly as including both symptomatic and prophylactic treatment, e.g., in the case of asthma, symptomatic treatment to ameliorate acute inflammatory events and prophylactic treatment to inhibit ongoing inflammatory status and to ameliorate future bronchial exacerbation associated therewith.

As used herein, the term "inflammatory diseases" refer to diseases which are a result of an individual's reaction of connecting tissue and blood vessels to an external or internal inflammation stimulus, with the purpose to eliminate or inactivate said stimulus. Triggering effectors include mechanical stimuli, or other physical factors, e.g. ionizing radiation, UV light, heat, coldness; chemical substances, e.g. bases, acids, heavy metals, bacterial toxins, allergens, and immune complexes, as well as pathogens, e.g. microorganisms and viruses, worms and insects; and pathogenic metabolism products, malfunctioning enzymes, malign tumors.

When inflammation occurs, chemicals, in particular so-called cytokines, from the body's white blood cells (leukocytes) are released into the blood or affected tissues to protect from foreign substances. This release of chemicals increases the blood flow to the area of injury or infection and may result in redness and warmth. Some of the chemicals cause a leak of fluid into the tissues, resulting in swelling. This protective process may stimulate nerves and cause pain. The expression of some of these chemicals/cytokines is regulated by NF-AT. The term "NF-AT" means "Nuclear Factor of Activated T cells". NF-AT belongs to a family of transcription factors, which regulate the expression of cytokine genes in T-lymphocytes, as described supra. Member of that family include NF-AT1. NF-AT2, NF-AT3 (also known as NF-AT4c), NF-AT4 and NF-AT5. Consequently, "inflammatory diseases" are preferably "NF-AT-dependent inflammatory diseases" refening to inflammatory diseases that arise due to an increased production of cytokines, the transcription of which is up-regulated by activated NF-AT transcription factors. Examples of such diseases are given below in greater detail.

As used herein, "enhancing the phosphorylation of NF-AT" means a higher phosphorylation grade of NF-AT as compared to NF-AT in blood cells which were not exposed to rocaglamide derivatives. "Higher" means at least 1.5-fold higher, at least 2-fold higher, at least 4-fold higher, at least 8-fold higher, at least 10-fold higher, at least 20-fold higher, at least 50-fold higher, at least 100-fold higher. A useful measure for the detection of an enhanced NF-AT phosphorylation is to investigate for the phosphorylation of p38 and JNK, both of which in turn are known to promote NF-AT nuclear export by selectively phosphorylating NF-AT proteins at the Ser-Pro (SP) sequences at the beginning of their SRR-1 region (see Example 13).

JNK1 phosphorylates NF-AT2 and NF-AT4, whereas p38 selectively targets NF-AT1 and NF-AT3. For JNK1, the proposed mechanism is that phosphorylation of the SPRIEIT calcineurin-docking site of NF-AT2 blocks the interaction of NF-AT2 with calcineurin.

As used herein, the term "leukocytes" encompasses T lymphocytes, i.e. both types of T helper cells (Th1 and Th2) and T killer cells, macrophages, granulocytes, and monocytes. In a preferred embodiment, "leukocytes" refers to Th1 and Th2 cells.

As used herein, "therapeutically effective amount" of one of the rocaglamide derivatives means a sufficient amount of the rocaglamide derivative to treat a particular disease, at a reasonable benefit/risk ratio. In general, the term "therapeutically effective amount" shall refer to an amount of the rocaglamide derivative which is physiologically significant and improves an individual's health. An agent, i.e. the rocaglamide derivative, is physiologically significant if its presence results in a change in the physiology of the recipient human. For example, in the treatment of a pathological condition, administration of the rocaglamide derivative which relieves or arrests further progress of the condition would be considered both physiologically significant and therapeutically effective. The rocaglamide derivatives may be employed in pure form or, where such forms exist, in pharmaceutically acceptable salt, ester or prodrug forms.

According to the present invention, the therapeutically effective amount refers to a rocaglamide dose which does essentially not inhibit the activity of NF-kappa B and/or AP-1.

"Essentially not" means in the present context that NF-kappa B and/or AP-1 are inhibited with the therapeutically effective amount of the rocaglamide derivative less than 50%, preferably less than 30%, more preferably less than 15%, most preferably less than 10%, and even most preferred less than 5% of the activity which is detected or measured in the absence of the rocaglamide derivative. In this respect, it is to be understood that rocaglamide derivatives at conventional doses, as known from the prior art, inhibit NF-kappa B activity induced by TNF-alpha which does not activate T cells to produce cytokines, whereas low Rocaglamide doses as disclosed in the present invention inhibit NF-AT activity in cells which were stimulated to produce cytokines via stimulation of the T cell receptor with, e.g. antibodies against CD3 and/or CD28.

In a preferred embodiment of the present invention, the therapeutically effective amount of the mcaglamide derivative inhibits the production of cytokines.

The term "cytokine" refers to proteins, produced and released predominantly by cells of the immune system, i.e. particularly T lymphocytes, macrophages, monocytes and mast cells. Cytokines serve as messengers for the immune cells and are responsible for the progress of an immune reaction. Cytokines, which promote inflammatory processes, include e.g. Tumor Nekrosis Factor (TNF)-α, IFN (Interferone)-γ, IL-1, IL-2, IL-6, IL-12, Macrophage-CoIony-Stimulating Factor (M-CSF) and Granulocyte/Macrophage-Colony-Stimulating Factor (GM-CSF). Cytokines, which inhibit an inflammatory response, include e.g. IL-10 and Transforming Growth Factor (TGF)-β. In a preferred embodiment of the present invention the cytokine is selected from the group consisting of IFN-γ, TNF-α, GM-CSF, IL-1, IL-2, IL-4, IL-5, IL-6, IL-8, and IL-12, more preferred IFN-γ, TNF-α, IL-2 and/or IL-4.

The inventors have found that higher effectives doses of rocaglamide derivatives, as these doses inhibit NF-kappa B activity, may also induce undesired programmed cell death (apoptosis). Thus, in a preferred embodiment of the present invention the therapeutically effective amount of the rocaglamide derivative comprises a blood plasma concentration in a range of from 15 nM to 100 nM, preferably of from 18 nM to 90 nM, more preferably of from 20 nM to 70 nM, most preferably of from 23 nM to 50 nM, and even most preferably of from 25 nM to 30 nM. It is to be understood that the total amount of the rocaglamide derivative may vary depending on the volume of blood plasma the medicament of the present invention is delivered to and depending on the physiological resorption of medicament ingredients by diverse tissues and organs. As an example: given an average molecular weight of rocaglamide derivatives of about 510g/mol and an average blood volume in an individual of 5-6 liter, a 15 nM concentration would equal a dose of a rocaglamide derivative to be administered of about 42 microgram (µg). But to achieve a 15 nM or higher blood plasma concentration the total amount is most likely much higher. Suitable means and measures, however, to determine the therapeutically effective amount, are available to the person skilled in the art. For example, distinct amounts of the rocaglamide derivative are administered to an individual and after various time points, the concentration of the rocaglamide derivative in said individual's blood plasma is determined.

As described supra, the rocaglamide derivatives are useful in the treatment of inflammatory diseases, particularly NF-AT dependent inflammatory diseases which may arise due to an increased cytokine production by NF-AT activation in response to an external or internal inflammation stimulus. Such inflammatory diseases can be subdivided into chronic inflammatory diseases, acute inflammatory diseases, allergic inflammatory disorders, graft-versus-host rejection diseases, and autoimmune disorders, all of which are encompassed within the present invention. Among these subgroups, inflammatory diseases of the respiratory tract, inflammatory skin diseases, allergic inflammatory disorders, inflammatory diseases of the gastrointestinal tract and inflammatory heart diseases can occur.

Accordingly, Rocaglamide derivatives are useful for the treatment of diseases or conditions responsive to or requiring anti-inflammatory, immunosuppressive or related therapy, including topical administration for the treatment of such diseases or conditions of the eye, nasal passages, buccal cavity, skin, heart, colon or, especially, airways or lung. In particular rocaglamide derivatives permit topical anti-inflammatory, immunosuppressive or related therapy with the concomitant avoidance or reduction of undesirable systemic side effects, for example renal toxicity or general systemic immunosuppression.

Rocaglamide derivatives are particularly useful for the treatment of diseases and conditions of the airways or lung, in particular inflammatory or obstructive airways disease. They are especially useful for the treatment of diseases or conditions of the airways or lung associated with or characterized by inflammatory cell infiltration or other inflammatory event accompanied by the accumulation of inflammatory cells, e.g. eosinophils and/or neutrophils.

In this respect, Rocaglamide derivatives are useful in the treatment of asthma of whatever type of genesis including both intrinsic and, especially, extrinsic asthma. They are useful for the treatment of atopic and non-atopic asthma, including allergic asthma, bronchitic asthma, exercise-induced asthma, occupational asthma, asthma induced following bacterial infection and other non-allergic asthmas. Rocaglamide derivatives are also useful for the treatment of bronchitis or for the treatment of chronic or acute airways obstruction associated therewith. Rocaglamide derivatives may be used for the treatment of bronchitis of whatever type or genesis, including, for example, acute bronchitis, arachidic bronchitis, catarrhal bronchitis, chronic bronchitis, croupous bronchitis, phthinoid bronchitis and so forth. Rocaglamide derivatives are in addition useful for the treatment of pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, berylliosis, chalicosis, ptilosis, siderosis, silicosis, tabacosis and, in particular, byssinosis. Rocaglamide derivatives may also be used to treat any disease or condition of the airways or lung requiring immunosuppressive therapy, e.g., for the treatment of autoimmune diseases of, or as they affect, the lungs (for example, for the treatment of sarcoidosis, alveolitis or chronic hypersensitivity pneumonitis) or for the maintainance of allogenic lung transplant, e.g., following lung or heart lung transplantation.

For the above purposes, some rocaglamide derivatives preferably will be administered topically within the airways, e.g. by the pulmonary route, by inhalation. While having potent efficacy when administered topically, rocaglamide derivatives are devoid of, or exhibit relatively reduced, systemic activity, e.g. following oral administration. Rocaglamide derivatives thus provide a means for the treatment of diseases and conditions of the airways or lung with the avoidance of unwanted systemic side effect, e.g., consequent to inadvertent swallowing of drug substance during inhalation therapy. (It is estimated that during the course of maneuvers required to effect administration by inhalation, up to 90% or more of total drug substance administered will inadvertently be swallowed rather than inhaled). By the provision of rocaglamide derivatives which are topically active, e.g. effective when inhaled but systemically inactive, the present invention makes rocaglamide derivative therapy available to subjects for whom such therapy might otherwise be excluded, e.g., due to the risk of systemic, in particular immunosuppressive, side effects.

Rocaglamide derivatives are also useful for the treatment of other diseases or conditions, in particular diseases or conditions having an autoimmune or inflammatory component and for which topical therapy may be practiced, for example, treatment of diseases and conditions of the eye such as conjunctivitis, keratoconjunctivitis sicca, and vernal conjunctivitis and maintenance of corneal transplant, diseases affecting the nose including allergic rhinitis, diseases and conditions of the skin including psoriasis, atopic dermatitis, pemphigus and contact dermatitis, as well as diseases of the colon, for example Crohn's disease and ulcerative colitis.

As immunosuppressants, rocaglamide derivatives are useful when administered for the prevention of immune-mediated tissue or organ graft rejection. Examples of transplanted tissues and organs which suffer from these effects are heart, kidney, liver, medulla ossium, skin, cornea, lung, pancreas, intestinum tenue, limb, muscle, nervus, duodenum, small-bowel, pancreatic-islet-cell, and the like; as well as graft-versus-host diseases brought about by medulla ossium transplantation.

The regulation of the immune response by rocaglamide derivatives would also find utility in the treatment of autoimmune disorders, such as rheumatoid arthritis, systemic lupus erythematosis, hyperimmunoglobulin E, Hashimoto's thyroiditis, multiple sclerosis, progressive systemic sclerosis, myasthenia gravis, type I diabetes, uveitis, allergic encephalomyelitis, glomerulonephritis, and the like; and further infectious diseases caused by pathogenic microorganisms, such as HIV. In the particular cases of HIV-1, HIV-2 and related retroviral strains, inhibition of T-cell mitosis would suppress the replication of the virus, since the virus relies upon the host T-cell's proliferative functions to replicate.

Further uses include the treatment and prophylaxis of inflammatory and hyperproliferative skin diseases and cutaneous manifestations of immunologically-mediated illnesses, such as psoriasis, atopical dermatitis, contact dermatitis and further eczematous dermatitises, seborrhoeis dermatitis, Lichen planus, Pemphigus, bullous pemphigoid, Epidermolysis bullosa, urticaria, angioedemas, vasculitides, erythemas, cutaneous eosinophilias, Lupus erythematosus, acne and Alopecia areata; various eye diseases (autoimmune and otherwise) such as keratoconjunctivitis, vernal conjunctivitis, keratitis, herpetic keratitis, conical cornea, dystrophia epithelialis comeae, corneal leukoma, ocular pemphigus, Mooren's ulcer, Scleritis, Graves' opthalmopathy, Vogt-Koyanagi-Harada syndrome, sarcoidosis, multiple myeloma, etc.; inflammation of mucosa and blood vessels such as gastric ulcers, vascular damage caused by ischemic diseases and thrombosis. Moreover, hyperproliferative vascular diseases such as intimal smooth muscle cell hyperplasia, restenosis and vascular occlusion, particularly following biologically- or mechanically-mediated vascular injury can be treated or prevented by rocaglamide derivatives.

Other treatable conditions would include but are not limited to ischemic bowel diseases, inflammatory bowel diseases, necrotizing enterocolitis, intestinal lesions associated with thermal burns and leukotriene B4-mediated diseases; intestinal inflammations/allergies such as Coeliac diseases, proctitis, eosinophilic gastroenteritis, and mastocytosis; food-related allergic diseases which have symptomatic manifestation remote from the gastrointestinal tract (e.g., migraine, rhinitis and eczema); renal diseases such as interstitial nephritis, Goodpasture's syndrome, hemolytic-uremic syndrome and diabetic nephropathy; nervous diseases such as multiple myositis, Guillain-Barre syndrome, Meniere's disease, polyneuritis, multiple neuritis, mononeuritis and radiculopathy; endocrine diseases such as hyperthyroidism and Basedow's disease; hematic diseases such as pure red cell aplasia, aplastic anemia, hypoplastic anemia, idiopathic thrombocytopenic purpura, autoimmune hemolytic anemia, agranulocytosis, pernicious anemia, megaloblastic anemia and anerythroplasia; bone diseases such as osteoporosis; respiratory diseases such as sarcoidosis, fibroid lung and idiopathic interstitial pneumonia; skin disease such as dermatomyositis, leukoderma vulgaris, ichthyosis vulgaris, photoallergic sensitivity and cutaneous T cell lymphoma; circulatory diseases such as arteriosclerosis, atherosclerosis, aortitis syndrome, polyarteritis nodosa and myocardosis; collagen diseases such as scleroderma, Wegener's granuloma and Sjogren's syndrome; adiposis; eosinophilic fasciitis; periodontal disease such as lesions of gingiva, periodontium, alveolar bone and substantia ossea dentis; nephrotic syndrome such as glomerulonephritis; male pattern aleopecia or alopecia senilis by preventing epilation or providing hair germination and/or promoting hair generation and hair growth; muscular dystrophy; Pyoderma and Sezary's syndrome; Addison's disease; active oxygen-mediated diseases, as for example organ injury such as ischemia-reperfusion injury of organs (such as heart, liver, kidney and digestive tract) which occurs upon preservation, transplantation or ischemic disease (for example, thrombosis and cardiac infraction): intestinal diseases such as endotoxin-shock, pseudomembranous colitis and colitis caused by drug or radiation; renal diseases such as ischemic acute renal insufficiency and chronic renal insufficiency; pulmonary diseases such as toxinosis caused by lung-oxygen or drug (for example, paracort and bleomycins), lung cancer and pulmonary emphysema; ocular diseases such as cataracta, siderosis, retinitis, pigmentosa, senile macular degeneration, vitreal scarring and corneal alkali burn; dermatitis such as erythema multiforme, linear IgA ballous dermatitis and cement dermatitis; and others such as gingivitis, periodontitis, sepsis, pancreatitis, diseases caused by environmental pollution (for example, air pollution), aging, carcinogenis, metastasis of carcinoma and hypobaropathy; disease caused by histamine or leukotriene-C4 release; Behcet's disease such as intestinal-, vasculo- or neuro-Behcet's disease, and also Behcet's which affects the oral cavity, skin, eye, vulva, articulation, epididymis, lung, kidney and so on.

Aqueous liquid compositions of the present invention may be particularly useful for the treatment and prevention of various diseases of the eye such as autoimmune diseases (including, for example, conical cornea, keratitis, dysophia epithelialis corneae, leukoma, Mooren's ulcer, sclevitis and Graves' ophthalmopathy) and rejection of corneal transplantation. In particular, compositions pertaining to the present invention are useful for treating a subject for immune-mediated organ or tissue allograft rejection, a graft-versus-host disease, an autoimmune disease, an obstructive airway disease, a hyperproliferative disease, or an ischemic or inflammatory intestinal or bowel disease.

Accordingly, the present invention further refers to pharmaceutical preparations for the treatment of inflammatory diseases which comprises a rocaglamide derivative of formula (I) and, optionally, a pharmaceutically acceptable carrier,
wherein the rocaglamide derivative of the formula (I) is used in a therapeutically effective amount which enhances the phosphorylation of NF-AT in leukocytes of a patient but which does essentially not inhibit NF-kappa B activity and AP-1 activity, and methods for preparing such pharmaceutical compositions. The methods for preparing pharmaceutical compositions, i.e. medicaments, are known *per se* to the skilled artisan.

The term "therapeutically effective amount" refers to the definition given supra. The rocaglamide derivatives may be employed in pure form or, where such forms exist, in pharmaceutically acceptable salt, ester or prodrug forms. Alternatively, the rocaglamide derivatives may be administered as pharmaceutical compositions containing the rocaglamide derivative of interest in combination with one or more drugs or pharmaceutically acceptable excipients. It will be understood, however, that the total daily usage of the rocaglamide derivatives and compositions of the present invention will be decided by the attending physician within the scope of sound medical judgment. As described above, the desired therapeutically effective amount of a rocaglamide derivative for use according to the present invention preferably does not inhibit NF-kappa B and/or AP-1 activity. Thus, the therapeutically effective amount preferably comprises a blood plasma concentration in a range of from 15 nM to 100 nM, preferably of from 18 nM to 90 nM, more preferably of from 20 nM to 70 nM, most preferably of from 23 nM to 50 nM, and even most preferably of from 25 nM to 30 nM.

The specific therapeutically-effective dose level for any particular patient will depend upon a variety of factors including the disorder being treated and the severity of the disorder; activity of the specific rocaglamide derivative employed; the specific composition employed; the age, body weight, general health, sex and diet of the patient; the time of administration, route of administration, and rate of excretion of the specific rocaglamide derivative employed; the duration of the treatment; drugs used in combination or coincidental with the specific rocaglamide derivative employed; and like factors well known in the medical arts. For example, it is well within the skill of the art to start doses of the rocaglamide derivative at levels lower than required to achieve the desired therapeutic effect and to gradually increase the dosage until the desired effect is achieved.

As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, phosphate buffer solutions; non-toxic, compatible lubricants such as sodium lauryl sulfate and magnesium stearate; as well as coloring agents, releasing agents, coating agents, sweetening, flavoring and perfuming agents. Preservatives and antioxidants can also be present in the composition, according to the judgment of the formulator.

The compositions may be administered to humans and other animals orally, rectally, parenterally, intracisternally, intravaginally, intraperitoneally, topically (as by powders, ointments, drops or transdermal patch), bucally, or as an oral or nasal spray. The term "parenteral" as used herein refers to modes of administration which include intravenous, intramuscular, intraperitoneal, intrasternal, subcutaneous and intraarticular injection and infusion.

Dosage forms for topical or transdermal administration of rocaglamide derivatives include ointments, pastes, creams, lotions, gels, plasters, cataplasms, powders, solutions, sprays, inhalants or patches. The active component, i.e. the rocaglamide derivative, is admixed under sterile conditions with a pharmaceutically acceptable carrier and any needed preservatives or buffers as may be required. The ointments, pastes, creams and gels may contain, in addition to an active rocaglamide derivative of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof. Powders and sprays can contain, in addition to rocaglamide derivatives, excipients such as lactose, talc, silicic acid, aluminum hydroxide, calcium silicates and polyamide powder, or mixtures of these substances. Sprays can additionally contain customary propellants such as chlorofluorohydrocarbons. For nasal administration, rocaglamide derivatives will suitably be administered in liquid or powdered form from a nasal applicator. Forms suitable for ophthalmic use will include lotions, tinctures, gels, ointment and ophthalmic inserts, again as known in the art. For rectal administration, i.e., for topical therapy of the colon, rocaglamide derivatives may be administered in suppository or enema form, in particular in solution, e.g., in vegetable oil or like oily system for use as a retention enema.

It is clear that safety may be maximized by delivering the drugs by the inhaled route either in nebuliser form or as dry powder. Clearly the great advantage of the inhaled route, over the systemic route, in the treatment of asthma and other diseases of airflow obstruction and/or of chronic sinusititis, is that patients are exposed to very small quantities of the drug and the rocaglamide derivative is delivered directly to the site of action.

Rocaglamide derivatives therefore are preferably employed in any dosage form appropriate for topical administration to the desired site. Thus, for the treatment of diseases of the airways or lungs, rocaglamide derivatives may be administered via the pulmonary route/by inhalation from an appropriate dispenser device. For this purpose, rocaglamide derivatives may be employed in any suitable finely dispersed or finely dispersible form, capable of administration into the airways or lungs, for example in finely divided dry particulate form or in dispersion or solution in any appropriate (i.e., pulmonarily administerable) solid or liquid carrier medium. For administration in dry particulate form, rocaglamide derivatives may, for example, be employed as such, i.e., in micronised form without any additive materials, in dilution with other appropriate finely divided inert solid carrier or diluent (e.g., glucose, lactose, mannitol, sorbitol, ribose, mannose or xylose), in coated particulate form or in any other appropriate form as known in the art for the pulmonary administration of finely divided solids. Pulmonary administration may be effected using any appropriate system as known in the art for delivering drug substance in dry or liquid form by inhalation, e.g. an atomizer, nebulizer, dry-powder inhaler or like device. Preferably a metered delivery device, i.e., capable of delivering a pre-determined amount of rocaglamide derivative at each actuation, will be employed. Such devices are known in the art.

Pharmaceutical compositions of this invention for parenteral injection comprise pharmaceutically-acceptable sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption, such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming mieroencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations may be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active rocaglamide derivative is mixed with at least one inert, pharmaceutically-acceptable excipient or carrier, such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules may be prepared with coatings and shells such as enteric coatings and other coatings well known in the pharmaceutical formulating art, They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Liquid dosage forms for oral administration include pharmaceutically-acceptable emulsions, solutions, suspensions, syrups and elixirs. In addition to the active rocaglamide derivatives, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Suspensions may contain, in addition to the active rocaglamide derivatives, suspending agents as, for example, ethoxylated isostearyl alcohols, polyoxyethylene sorbitol and sorbitan esters, microciystalline cellulose, aluminum metahydroxide, bentonite, agar-agar, and tragacanth, and mixtures thereof.

Topical administration includes administration to the skin or mucosa, including surfaces of the lung and eye. Compositions for topical administration, including those for inhalation, may be prepared as a dry powder which may be pressurized or non-pressuhzed. In non-pressurized powder compositions, the active ingredient in finely divided form may be used in admixture with a larger-sized pharmaceutically-acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers. Alternatively, the composition may be pressurized and contain a compressed gas, such as nitrogen or a liquified gas propellant. The liquified propellant medium and indeed the total composition are preferably such that the active ingredient does not dissolve therein to any substantial extent. The pressurized composition may also contain a surface-active agent, such as a liquid or solid non-ionic surface-active agent or may be a solid anionic surface-active agent. It is preferred to use the solid anionic surface-active agent in the form of a sodium salt.

A further form of topical administration is to the eye, as for the treatment of immune-mediated conditions of the eye such as autoimmune diseases, allergic or inflammatory conditions, and corneal transplants. The rocaglamide derivative is delivered in a pharmaceutically acceptable ophthalmic vehicle, such that the rocaglamide derivative is maintained in contact with the ocular surface for a sufficient time period to allow the rocaglamide derivative to penetrate the corneal and internal regions of the eye, as for example the anterior chamber, posterior chamber, vitreous body, aqueous humor, vitreous humor, cornea, iris/cilary, lens, choroid/retina and sclera. The pharmaceutically acceptable ophthalmic vehicle may, for example, be an ointment, vegetable oil or an encapsulating material.

Rocaglamide derivatives may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming Liposomes can be used. The present compositions in liposome form can contain, in addition to a rocaglamide derivative, stabilizers, preservatives, excipients, and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N.Y. (1976), p. 33 et seq.

As described supra in the present invention, the NF-AT dependent inflammatory diseases which may be treated by the low dosage use of rocaglamide derivatives, respectively the medicaments containing them, may arise due to an increased production of certain, NF-AT-regulated cytokines.

Therefore, the present invention further refers to a method of inhibiting cytokine production in cells, the method comprising the step of treating said cells with a rocaglamide derivative of the formula (I), wherein the rocaglamide derivative is applied to said cells in an effective amount which enhances in said cells the phosphorylation of NF-AT and which does essentially not inhibit NF-kappa B activity and AP-1 activity.

The method can be performed both *in vitro* and *in vivo*. In a preferred embodiment, the method is performed *in vitro*.

The term "cytokine" refers to the definition given supra. In a preferred embodiment of the present invention the cytokine is selected from the group consisting of IFN-γ, TNF-α, GM-CSF, IL-1, IL-2, IL-4, IL-5, IL-6, IL-8, and IL-12, more preferred IFN-γ, TNF-α, IL-2 and/or IL-4.

According to the invention, the effective amount which enhances in said cells the phosphorylation of NF-AT and which does essentially not inhibit NF-kappa B activity and AP-1 activity, comprises a concentration in a range of from 15 nM to 100 nM preferably of from 18 nM to 90 nM, more preferably of from 20 nM to 70 nM, most preferably of from 23 nM to 50 nM, and even most preferably of from 25 nM to 30 nM.

The present invention also relates to method(s) of treatment of inflammatory diseases or prevention of organ transplant rejection in a subject by administering to the subject therapeutically effective amounts of the rocaglamide derivatives with or without the concurrent use of other drugs or pharmaceutically acceptable excipients, as described throughout the present specification. The methods of the present invention comprise treating a subject in need of immunosuppresive, anti-inflammatory, antimicrobial, antifungal, antiviral or antiproliferative therapy, or requiring the reversal of chemotherapeutic drug resistance, by administering a therapeutically effective amount of a rocaglamide derivative for such time and in such amounts as is necessary to produce the desired result. Dosages of rocaglamide derivatives employed in practicing the method(s) of the present invention will of course vary depending on the site of treatment, the particular condition to be treated, the severity of the condition, the subject to be treated (e.g. in terms of body weight, age and so forth) as well as the effect desired.

In particular, the present invention refers to a method of treatment of an inflammatory disease in a patient, the method comprising the step of administering to said patient a rocaglamide derivative of the formula (I), wherein the rocaglamide derivative is administered in a therapeutically effective amount which enhances in said cells the phosphorylation of NF-AT and which does essentially not inhibit NF-kappa B activity and AP-1 activity.

### DESCRIPTION OF THE DRAWING

Fig. 1. Chemical structures of the three rocaglamide derivatives used in this study.
Fig. 2. Rocaglamides inhibit cytokine production in primary peripheral human T cells.
   Freshly isolated human peripheral blood T cells were stimulated with αCD3/αCD28 antibodies in the presence or absence of different concentration of rocaglamides (added 1 h before stimulation) as indicated. After 24 h stimulation, the supernatants were analysed for cytokine production by ELISA. Data are representative of six separate experiments with T cells from different donors.

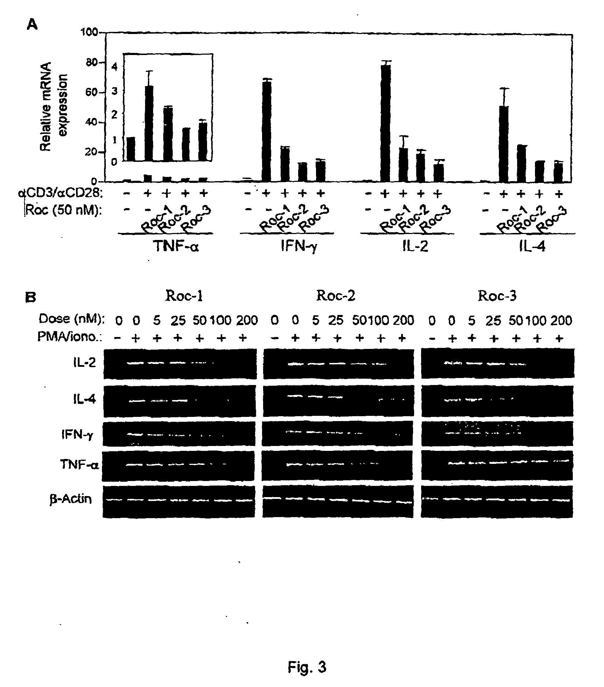

Fig.3. Rocaglamides inhibit cytokine mRNA expression in activated T cells.
   (A) Freshly isolated peripheral blood T cells were treated with 50 nM rocaglamides for 1 h and then stimulated with αCD3/αCD28 antibodies. After 2 h stimulation, total RNA was prepared and analysed for cytokine mRNA expression levels by real-time PCR. Results were internally confirmed by the comparative cycle count (Ct) against β-actin as the standard gene. One representative experiment (in triplicate PCR) of two is shown.
   (B) Jurkat T-cells were treated with different doses of rocaglamides for 1 h and then stimulated with PMA/ionomycin for 2 h. Total RNA was prepared and analyzed for cytokine mRNA expression levels by RT-PCR. β-actin mRNA expression levels were analysed as controls. Data are a representative of two separate experiments.

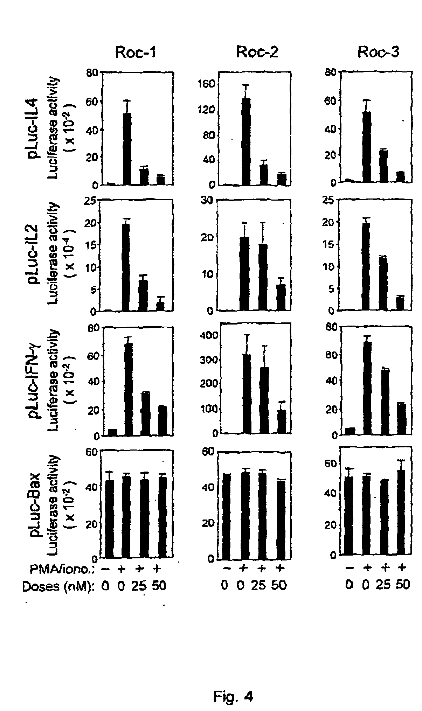

Fig. 4. Rocaglamides down-regulate promoter activities of cytokine genes.
   The promoter luciferase constructs containing the human IL-2, IL-4 and IFN-g gene were transfected into Jurkat T cells and after overnight culture, the cells were split and pre-incubated with the indicated amounts of rocaglamides or the rocaglamides solvent for 1 h and then stimulated with PMA/ionomycin or left unstimulated for 8 h. The promoter of a non-cytokine gene (Bax) was used as a control. The promoter activities are given as luciferase activity. One representative experiment (in triplicate transfections) of three independent experiments is shown.

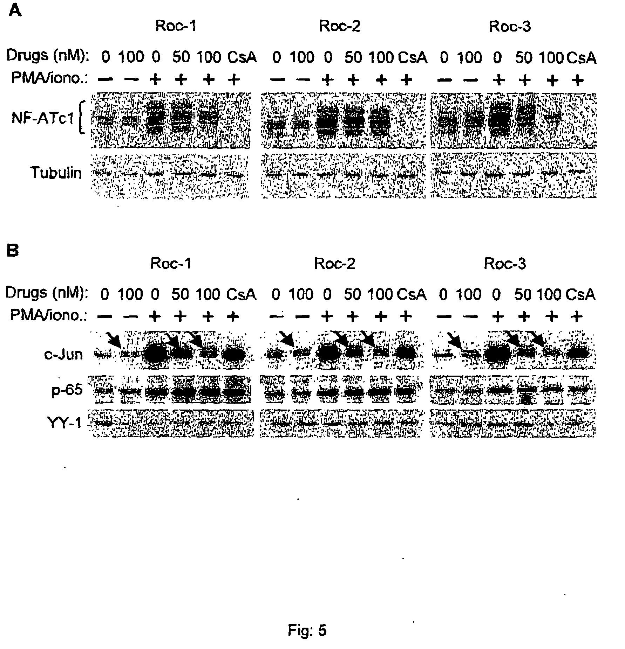

Fig. 5. Rocaglamides down-regulate T-cell-activation-induced nuclear expression of NF-AT and c-Jun,
   (A) Jurkat T cells were stimulated with PMA and ionomycin for 2 h in the absence or presence of different concentrations of rocaglamides (added 1 h before stimulation). Nuclear proteins were isolated and were immunoblotted with the monoclonal antibody against NF-ATcl. The α-NF-AT blot was stripped for innmunoblot to α-tubulin. (B) The nuclear proteins were immunobIotted with antibodies to c-Jun and p65 and then stripped for immunoblot to α-YY-1. Data are representative of three separate experiments.

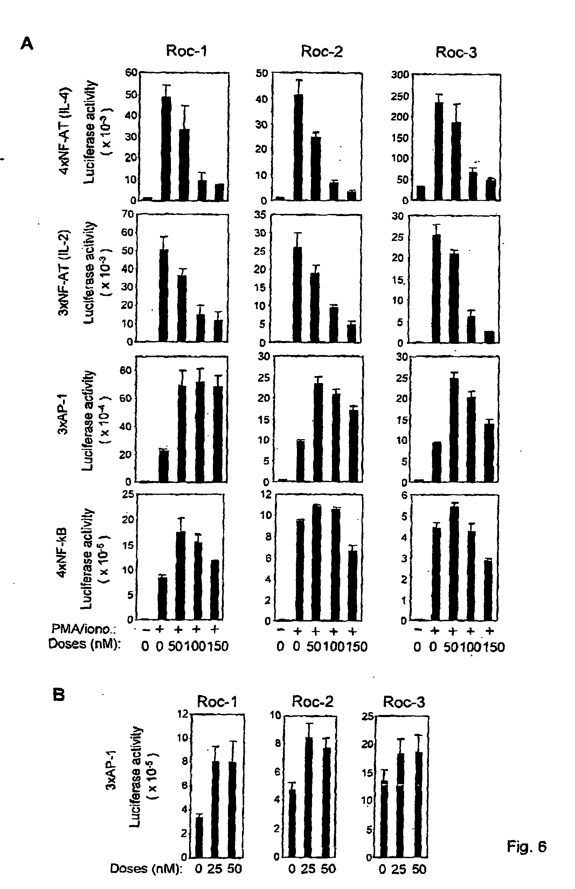

Fig. 6.Rocaglamides inhibit transcriptional activity mediated by the NF-AT elements derived from the IL-2 and IL-4 promoter.
   (A) Jurkat T cells were transfected with the luciferase reporter constructs containing multiple copies of the NF-AT elements derived from the IL-2 and IL-4 promoter, the consensus DNA-binding elements of AP-1 and NF-kappa B. After overnight culture, the cells were split and pre-incubated with the indicated amounts of rocaglamides or the rocaglamides solvent for 1 h and then stimulated with PMA/ionomycin or left unstimulated. Luciferase activity was determined 8 h post-stimulation. Data are representative of four independent experiments in triplicates.
   (B) Jurkat T cells were transfected with the luciferase-AP-1 reporter construct and after overnight recovery the cells were further cultured with the indicated amounts of rocaglamides. Luciferase activity was determined 8 h later. Data are representative of three independent experiments in triplicates.

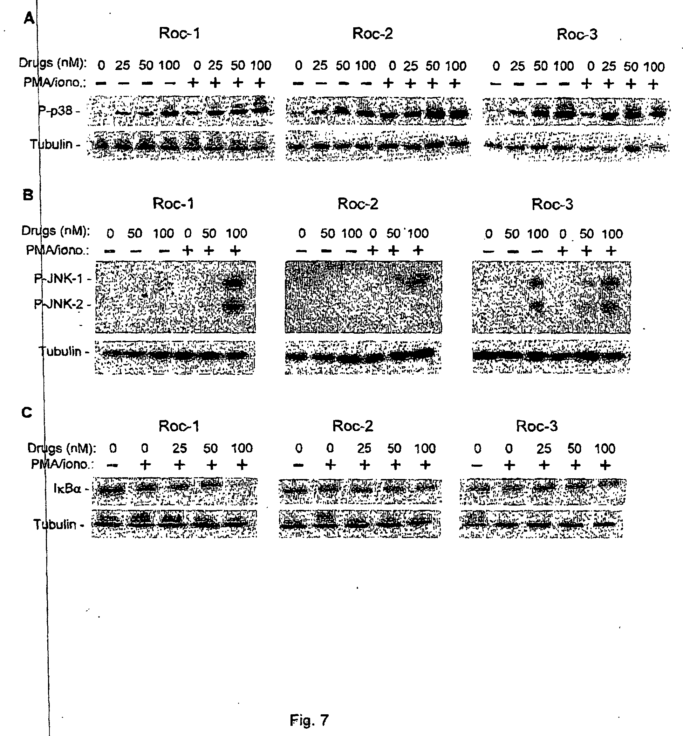

Fig. 7. Rocaglamides activate MAP kinases p38 and JNK.
   (A) Jurkat T cells were stimulated with PMA and ionomycin for 2 h in the absence or presence of different concentrations of rocaglamides (added 1 h before stimulation). Total cell lysates were immunoblotted with antibodies against phosphorylated p38 (p-p38) and was then stripped for immunoblot to α-tubulin.
   (B) Total cell lysates were immunoblotted with antibodies to phosphorylated JNK and then stripped for immunoblot to α-tubulin.
   (C) Total cell lysates were immunoblotted with antibody to I-kappa Bα and then stripped for immunoblot to α-tubulin. All data are representative of 2-3 reproducible experiments.

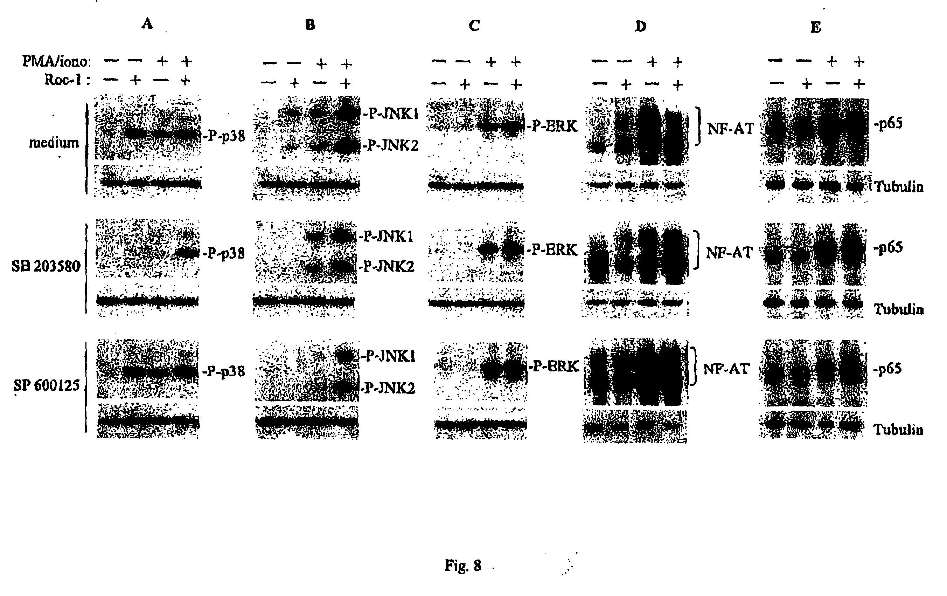

Fig. 8. Inhibitors of p38 and JNK prevent rocaglamide-induced reduction of nuclear NF-AT.

PMA/ionomyrin-activated Jurkat T cells were treated with 100 nM Roc-1 in the presence or absence of the p38 and JNK kinase inhibitors SB 203580 (5 µM) and SP 600125 (20 µM), respectively. (A), (B) and (C) Total lysates were immunoblotted with antibodies against phosphorylated p38, JNK and ERK, respectively and then stripped for immunoblot to α-tubulin, (D) and (E) Nuclear exptracts were immunoblotted with antibodies against NF-AT and p65, respectively and then stripped for immunoblot to α-tubulin.

Without being limited, the invention is now further illustrated by the following examples:

### EXAMPLES:

### Example 1: Cells and Cell culture

The Jurkat T leukemia cells were cultured in RPMI 1640 medium (GIBCO laboratories, Grand Island, NY) supplemented with 10% FCS, 50 µg/ml gentamicin (GIBCO), 6 mM HEPES (CCFBCO, 1 M solution), and 2 mM L-glutamine (GIBCO, 200 mM solution) at 37°C and 5% CO₂.

### Example 2: Rocaglamide derivatives

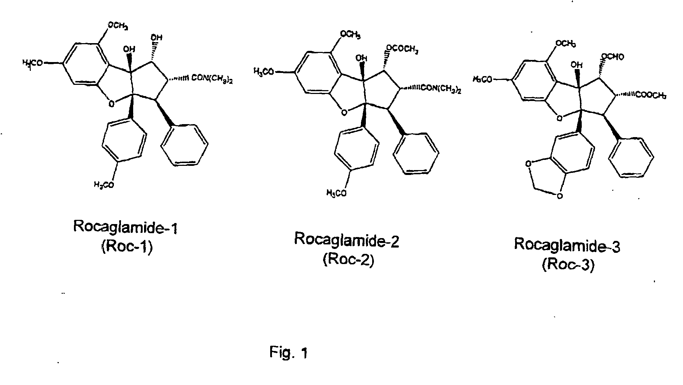

Rocaglamide derivatives used in this study (Fig. 1) had been isolated previously from various *Aglaia* species as reported in the literatures (e.g. Nugroho et a1.,1999; Schneider et al.,1999). The structures of the compounds were unequivocally elucidated based on their NMR and mass spectra as described before.

### Example 3: Purification of T-lymphocytes and apoptosis measurement

Human peripheral blood mononuclear cells were prepared by Ficoll-Paque (Pharmacia, Uppsala, Sweden) density centrifugation. Adherent cells were removed by adherence to plastic culture vessels for 1 h. T cells were isolated from the peripheral blood mononuclear cells by resetting with 2 amino-ethylisothyo-uronium-bromide treated sheep red blood cells (Klas et al., 1993). Apoptotic cell death was assessed by propidium iodide (PI) uptake and analyzed by FACS (Klas et al., 1993).

### Example 4: Determination of IL-4, IL-2, TNFα and IFN-γ proteins

Freshly isolated peripheral blood T cells (1x10⁶/ml) were stimulated with with plate-bound αCD3 (OKT3 10 µg/ml) and αCD28 (5 µg/ml) antibodies for 24 h. Supernatants were tested for the presence of IL-4, IL-2, TNF-α and IFN-γ using an enzyme-linked-immunosorbent-assay (ELISA) specific for human IL-4, EL-2, TNF-α and IFN-γ proteins (Pharmingen, Becton Dickinson Company, Belgium) according to the manufacturer's instruction.

### Example 5: Preparation of nuclear proteins, total cell lysates and immunoblotting

T cells were lysed in ice-cold RIPA buffer (50 mM Tris-HCl, pH 8.0, 120 mM NaCI, 1% NP-40, 0.5% Desoxycholate, 1 mM PMSF, 25 mM NaF, 1% SDS, 1 mM DTT, 0.2 mM Na₃V0₄ and 20 µl/ml protease inhibitors) for 30 min. Nuclear proteins were isolated as described previously (Li-Weber et al., 1998). The widely used p38 and JNK kinase inhibitors SB 203580 and SP 600125 were purchased from Alezis Biochemicals (Grünberg, Germany). Equal amounts of proteins were separated on 10% SDS-PAGE gels, transferred onto Hybond-ECL nitrocellulose membrane (Amersham Biosciences, Little Chalfon, UK). The membrane was blocked with 5% milk powder in PBS/0.1% Tween 20 for 1 h, washed with the same solution and incubated with antibodies at 37°C for 1 h or overnight at 4 °C. The blots were washed with PBS/Tween and developed with HRP-coupled antibodies followed by enhanced Chemiluminescence Reagent Plus (PerkinElmer Life Sciences, Boston, MA). The following antibodies were used: the antibodies against NF-kappa B p65 (A, sc-109) and 1-kappa Bα (C21, sc-371) were purchased from Santa Cruz Biotechnology, Inc. (Santa Cruz, CA); the anti-c-Jun monoclonal antibody from BD-Bioscience-Phamingen (Europe, Belgium); the anti-phospho-1-kappa Bα (Ser32) and anti-phospho-ERK antibodies from Cell Signalling (Beverly, MA); the anti-active p38 antibody from Promega (Heidelberg, Germany) and the anti-NF-ATc1 monoclonal antibody (7A6) from Alexis Biochemicals (Grünberg, Germany). For stripping, membranes were incubated for 30 min at 56 °C in a buffer containing 62.5 mM Tris-HCl, pH 6.7, 2% SDS and 100 mM β-mercaptoethanol.

### Example 6: RNA isolation and RT-TCR

Total cellular RNA was isolated using the RNeasy-kit (Qiagen, Hilden, Germany) according to the manufacturer's instructions. 1 µg RNA was reverse transcribed with oligo dT. PCR amplification was then performed for 30-35 cycles with specific primers (Stratagene Inc, Heidelberg, Germany) for human IL-2 (457 bp PCR product), human IL-4 (456 bp PCR product), human IFN-γ (501 bp PCR product) and human β-actin (661 bp PCR product) as previously described (Li-Weber et al., 1998). The PCR products were then subjected to agarose gel electrophoresis.

### Example 7: Quantitative real-time PCR

The principle of TagMan quantitative real-time PCR has previously been described in detail (Heid et al., 1996). The sequence for primers of IL-2, IL-4, IFN-γ, β-actin and fluorescent-labeled probes used in these studies was described previously (Li-Weber et al., 2002). The primers and probe of IFN-α is forward 5'-GGAGAAGGGTGACCGACTCA-3', reverse 5'-TGCCCAGACTCGGCAAG-3', and probe 5'-CGCTGAGATCAATCGOCCCGACTA-3'. PCR was performed in a 12.5 µl reaction mixture (PCR kit from Eurogentech, Belgian) that contained 0.08 µg of reverse transcribed cDNA and proper amount of primers and probe. For each sample three PCR reactions were performed. The resulting relative increase in reporter fluorescent dye emission was monitored by the TagMan-system (GeneAmp 5700 sequence detection system and software, Perkin Elmer, Foster City, CA). The level of the cytokine mRNA, relative to β-actin, was calculated using the formula: Relative mRNA expression = 2 ^{-(Ct of cytokine-Ct of β-actin)} where Ct is on the threshold cycle value.

### Example 8: Plasmid constructs and transient transfections

Luciferase reporter construct containing the human IL-2 (-300/+47), the human IL-4 (-269/+11) promoter and the multiple IL-4 NF-AT (P1) luciferase constructs were generated previously (Ehret et al., 2001; Li-Weber et al., 1998). The multiple copies of the AP-1 binding site from SV40 enhancer (CGGTTGCTGACTAATTG), the NF-kappa B consensus sequence (GGAAATTCCCC) and the human IL-2 NF-AT (-282/-250) element (GAAAGCAGGAAAAACTGTTTCATACAGAAGGC) were constructed by ligation of the DNA sequence into the multiple-cloning site of pTATA-Luc vector. The luciferase reporter construct containing the human IFN-γ (-854/+7) promoter was construct by ligation of the IFN-γ promoter sequence generated by PCR into the luciferase reporter vector. All constructs were confirmed by sequencing analysis. The pLuc-Bax promoter reporter plasmid was kindly provided by M. L. Schmitz (Department for Chemistry and Biochemistry, University of Bern, Switzerland).

Jurkat T cells were transfected by eleclroporation as previously described (Li-Weber et al., 1998). After overnight recovering, the cells were divided and treated with different doses of rocaglamides and than further cultured in the absence or presence of PMA (10 ng/ml) and ionomycin (0.5 µM) for 8 h. Luciferase activity was determined in 10 µl of cell extract using the luciferase assay substrate (Promega Corp., Heidelberg, Germany) with a Duolumat LB9507 luminometer (Berthold, Bad Wildbad, Germany).

### Example 9: Rocaglamides inhibit cytokine production in peripheral blood T cells

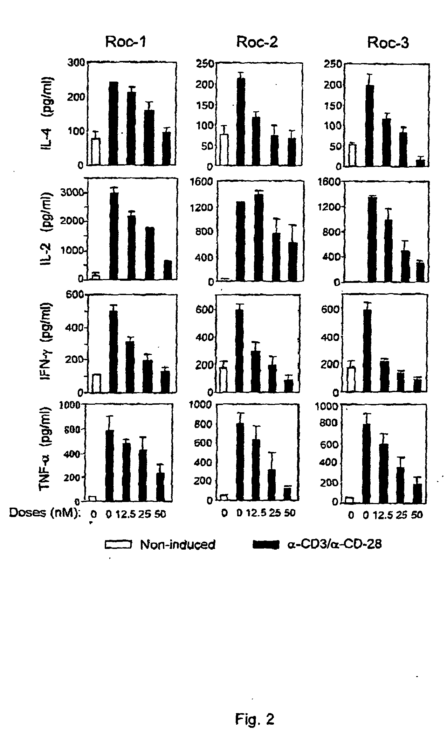

Three rocaglamide derivatives (named Roc-1, 2 and 3) were tested for their ability to inhibit expression of cytokine genes in T cells. These compounds differed mainly in the substitution patterns on the aromatic rings (Fig. 1) and were prepared to at least 98% purity as determined by high performance liquid chromatography (Schneider et al., 2000). To investigate whether rocaglamide derivatives influence cytokine production in T cells, freshly isolated human peripheral blood T cells were activated by either cross-linking of the TCR-CD3 molecules with α-CD3 plus a co-stimulation signal provided by α-CD28 antibodies or with phorbol-12-myristate-13-acetate (PMA) and ionomycin that mimic the antigen signals in the presence or absence of rocaglamides, The protein production levels of IFN-γ, TNF-α, IL-2 and IL-4 were determined 24 h post-stimulation by ELISA. Stimulation of T cells via TCR resulted in high levels of cytokine production. However, in the presence of rocaglamides, the production of IFN-γ, TNF-α, IL-2 and IL-4 was suppressed in a dose-dependent manner (Fig. 2). At the concentration of 50 nM, rocaglamides completely blocked T-cell-activation-indueed expression of IL-4 and IFN-γ and led to a 60-85% reduction in IL-2 and TNF-α production. At this dose, rocaglamide was not toxic to T cells. These data indicate that rocaglamides may suppress T cell activation and down-regulate cytokine gene expression.

### Example 10: Rocaglamides suppress cytokine mRNA expression in T cells

We next analyzed the effect of rocaglamides on cytokine expression in peripheral blood T cells at the mRNA level by quantitative real-time PCR. Consistent with the results obtained by ELISA, mRNA analysis showed that at the concentration of 50 nM rocaglamides significantly suppressed IFN-γ, TNF-α, IL-2 and IL-4 mRNA expression in peripheral blood T cells (Fig. 3A). We further investigated the molecular mechanisms by which rocaglamids suppress production of cytokines using the human leukemic T cell line, Jurkat, as a model system. Jurkat T cells express mRNAs of IFN-γ, TNF-α, IL-2 and IL-4, and are often used instudies of various cytokine genes. Cytokine mRNA expression levels in Jurkat T cells were analyzed by reverse transcriptase (RT)-PCR. Similar to the results obtained with the peripheral blood T cells, rocaglamides suppressed mRNA expression of all four cytokine genes tested in a dose-dependent fashion (Fig. 3B). To further investigate whether suppression of mRNA expression by rocaglamides occurred at the transcriptional level, luciferase reporter plasmids containing promoters of the human IFN-γ, IL-2 and IL-4 gene were subjected to transient transfection studies. Transfected Jurkat T cells were stimulated by PMA and ionomycin in the presence or absence of different doses of rocaglamides. In agreement with the expression levels of the endogenous mRNAs, the promoter activities of the three cytokine genes tested were down-regulated by rocaglamides in a dose-dependent manner (Fig. 4). In the control experiment, activity of a promoter reporter construct containing a constitutive non-cytokine gene Bax was not influenced by rocaglamides. These experiments demonstrate that rocaglamide derivatives may directly inhibit cytokine gene expression at the transcriptional level.

### Example 11: Rocaglamides inhibit nuclear expression of NF-AT and c-Jun

Many inflammatory cytokine genes are transcriptionally activated by the inducible, ubiquitous transcription factors NF-AT, AP-1 and NF-kappa B (Tsai et al., 1996; Sica et al., 1997; Falvo et al., 2000; Macian et aL, 2001; Kaminuma et al., 2002; Li-Weber & Krammer, 2003). Therefore, we first examined the effect of the rocagtamide derivatives on nuclear expression levels of NF-AT, the AP-1 subunit c-Jun, and the NF-kappa B subunit p65 in activated T cells. Nuclear proteins were prepared from Jurkat T cells, stimulated by PMA/iouomycin for 2 h in the presence or absence of different doses of rocaglamides. Immunoblotting analysis of nuclear levels of these transcription factors showed a dose-dependent reduction of the T-cell-activation-induced nuclear expression of NF-AT and c-Jun (Fig. 5). CsA is a well known immunosuppressive reagent that targets NF-AT activity. In comparison, CsA strongly inhibits NF-AT but only moderately influences c-Jun nuclear expression (Fig. 5B). Previously, certain rocaglamide derivatives, e.g. Roc-1 (previously named as Roc-4), were shown to inhibit NF-kappa B activity induced by TNF-α or PMA (Baumann et al., 2002). At the doses used in our experiments, no reduction in the nuclear levels of p65 was seen. Instead, the nuclear level of p65 was slightly elevated in Roc-1-treated cells (Fig. 5B). As controls, rocaglamides did not influence protein levels of tubulin or the constitutively expressed transcription factor YY-1. These data indicate that rocaglamides may differentially impair NF-AT and c-Jun expression in activated T cells.

### Example 12: Rocaglamides inhibit NF-AT but not c-Jun and NF-kappa B activity

Characteristically, NF-AT family proteins act synergistically with AP-1 (Fos/Jun) proteins to activate genes containing composite NF-AT and AP-1 binding sites (Macian et al., 2001). Many cytokine genes, including IL-2, IL-4, and IFN-γ contain NF-AT/AP-1 composite sites in their promoter/enhancer region (Rao et al., 1997). To investigate whether inhibition of NF-AT and c-Jun expression by rocaglamides would lead to suppression of their transcriptional activities, we performed transfection studies with luciferase reporter constructs containing multiple copies of the NF-AT-binding elements derived from the IL-4 and IL-2 promoter and the consensus binding sequences for AP-1 and NF-kappa B, respectively. As expected, in the presence of rocaglamides transcriptional activities mediated by the IL-4 or the IL-2 NF-AT elements were down-regulated in a dose-dependent manner (Fig. 6A). Consistent with the immunoblotting data, rocaglamides had no inhibitory effect on NF-kappa B activity at concentrations up to 100 nM. By contrast, a subtle increase in NF-kappa B activity was seen in cells treated with Roc-1 (Fig. 6A). Moderate inhibition (about 20%) of NF-kappa B-dependent transcription was seen in cells treated with Roc-2 and Roc-3 at doses higher than 150 nM. At the doses higher than 200 nM, all three rocaglamide derivatives inhibited NF-kappa B activity to a variable degree (data not shown). Unexpectedly, AP-1-mediated transcriptional activity was substantially enhanced at concentrations of rocaglamides that suppressed NF-AT activity (Fig. 6A). We noticed that rocaglamide treatment induced phosphorylation of c-Jun (Fig. 5B, indicated by arrows). Since c-Jun activity can be enhanced by phosphorylation (Johnson GL & Lapadat R. 2002), we assume that rocaglamides may increase AP-1-dependent transcription via phosphorylation of c-Jun, Indeed, treatment of Jurkat T cells with rocaglamides alone led to a substantial increase in AP-1-dependent gene expression (Fig. 6B).

### Example 13: Rocaglamides inhibits NF-AT via activation of the MAP kinases JNK and p38

The above experiments showed that at doses ranging from 25-100 nM rocaglamides selectively inhibited NF-AT function without impairing activities of AP-1 and NF-kappa B. NF-AT family of proteins are activated by the calcium/calmodulin-dependent phosphatase calcineurin that dephosphorylates and promotes nuclear translocation of NF-AT (Macian et al., 2001; Crabtree et al., 2002). Glycogen synthase kinase-3 (GSK3) is the constitutive NF-AT kinase which induces rephosphorylation and, therefore, inactivation of NF-AT (Beals et la., 1997; Graef et al., 1999). Inactivation of NF-AT may also be regulated by the cellular MAP kinases JNK and p38 that rephosphorylate NF-AT (Gomez et al., 2000; Porter et al., 2000; Chow et al., 2000). To investigate the molecular mechanisms of rocaglamides-mediated suppression of NF-AT activity, we examined the effect of rocaglamides on activities of MAP kinases. We found that treatment with rocaglamides alone induced p38 phosphorylation in Jurkat T cells. At a concentration of 25 nM, rocaglamides induced phosphorylation of p38 similar to that observed by PMA and ionomycin (Fig. 7A). In PMA/ionomycin-stimulated Juarkat T cells, rocaglamides further enhanced T-cell-activation-induced phosphorylation of p38 (Fig. 7A). Rocaglamides also synergize with PMA/ionomycin to induce expression of phosphor-JNK (p-JNK) (Fig. 7B). Subtle differences in their ability to induce JNK phosphorylation were observed between the three rocaglamide derivatives. Roc-1 and 3 are stronger inducers of p-JNK than Roc-2. Consistent with the immunoblotting and transfection studies, rocaglamides did not inhibit PMA/ionomycin-induced degradation of I-kappa B at the concentrations 25-100 nM (Fig. 7C).

To further investigate whether activation of MAP kinases by rocagiamides is the cause of reduction in NF-AT activity, PMA/ionomycin-activated Jurkat T cells were treated with rocaglamides in the presence or absence of the widely used p38 and JNK kinase inhibitors SB 203580 and SP 600125, respectively. Immunoblotting analysis showed that rocaglaznide-enhanced phosphorylation of p38 and JNK was largely inhibited in the presence of such specific inhibitors (Fig. 8A and B). In contrast, rocaglamide does not induce phosphorylation of ERK and the p38 and JNK inhibitors have no effect on ERK activities (Fig. 8C). Inhibition of p38 and JNK phosphorylation by the inhibitors correlated with prevention of rocaglamide-mediated reduction in NF-AT proteins in the nucleus (Fig. 8D). As control, the nuclear levels of the NF-kappa B subunit p65 were not influenced by the doses of rocaglamide used in the same experiments. In addition, they were also not affected by the p38 or the JNK kinase inhibitors (Fig. 8E). Thus, inhibition of p38 and JNK activity prevents rocaglamide-mediated inhibition of NF-AT nuclear expression. These data demonstrate that rocaglamides-mediated inhibition of NF-AT activity is associated with over-activation of JNK/p38 kinases.

### Example 14: Rocaglamide derivatives as therapeutic drugs for the treatment of NFAT-dependent diseases

The NF-AT family of transcription factors encompasses five evolutionarily related proteins: NF-AT1 to NF-AT5. Except NF-AT5, activation of NF-AT1 to NF-AT4 is regulated by Ca²⁺ and Ca²⁺/calmodulin-dependent serine phosphatase calcineurin. Mature T cells express predominantly NFAT1 and NFAT2. NFAT1 and NFAT2 activate transcription of a large number of cytokine genes including IFN-γ, TNF-α, and IL-4 during an effective immune response. However, uncontrolled expression these cytokines is dangerous and causes diseases such as rheumatoid arthritis, diabetes, hepatitis, asthma, rhinoconjunctivities and anaphylaxis. NFAT activities are also critically involved in diseases such as allograft rejection and graft-versus-host disease.

NF-AT signaling also plays a role in cardiac valve development. Mice bearing deletions in NF-AT3 and NF-AT4 die with vasculature patterning defects. In addition, evidence shows that NF-AT3 (also called NF-ATc4) is responsible for cardiac hypertrophy. Transgenic mice that express activated forms of NF-AT3 in the heart develop cardiac hypertrophy and heart failure that mimic human heart disease. Drugs such as Cyclosporin A (CsA) and FK506 can prevent myocyte hypertrophy *in vitro* and can block cardiac hypertrophy in vivo in response to numerous stimuli including pressure overload, myocardial infarction, hypertension, and some abnormalities in contractile proteins. However, the level of calcineurin, expression in the heart is approximately 10-fold higher than in the immune system, making it unlikely that CsA or FK506 could be used chronically at sufficient doses to treat cardiac disease without immune suppression and secondary renal toxicity.

CsA and FK506 suppress NF-AT activity via inhibition of calcineurin-mediated NF-AT dephosphorylation and thereby prevent NFAT nuclear translocation (Hogan et al., 2003, Genes Dev., Vol. 17, pages 2205-2232). Several constitutive kinases such as CK1 and GSK3 and inducible kinases such as the MAP kinases p38 and JNK can phosphorylate NF-AT and promote NF-AT nuclear export (Hogan et al., 2003, see supra). We show that rocaglamides inhibit NF-AT activity via over-activation of p38 and JNK. JNK-1 phosphorylate NF-AT2 and NFAT4, whereas p38 selectively targets NF-AT1 and NF-AT3. Since rocaglamides inhibit NF-AT activity through a mechanism different from that CsA and FK506 the use of rocaglamides is a new and inventive tool for the treatment of cardiac disease.

## Claims

1. Use of a rocaglamide derivative of the formula (I) 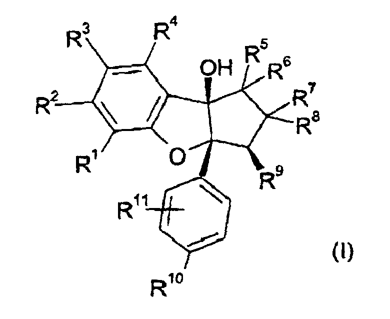 wherein
R¹ is selected from hydrogen, halogen and alkyl;
R² is selected from halogen, alkyl and alkoxy;
R³ is selected from hydrogen, halogen and alkyl;
R⁴ is selected from halogen, alkyl and alkoxy;
or R² and R³ together form a -OCH₂CH₂O- unit;
R⁵ is selected from hydroxyl, acyloxy, amino, monoalkylamino, dialkylamino and - NR¹²-CHR¹³-COOR¹⁴, with
R¹² being selected from hydrogen and alkyl,
R¹³ being selected from phenyl and benzyl, which both may carry a substituent from the group hydroxyl, indolyl and imidazolylmethyl, and alkyl which may be substituted by a group selected from OH, SH, alkoxy, thioalkoxy, amino, monoalkylamino, dialkylamino, carboxy, carboxyallryl, carboxamide and hydroxyl groups;
or R¹² and R¹³ together form a -(CH₂)₃- or -(CH₂)₄- group;
R¹⁴ being selected from alkyl and benzyl; in which case R⁶ is hydrogen, or
R⁵ and R⁶ together form an oxo or hydroxyimino group;
R⁷ is hydrogen;
R⁸ is selected from hydrogen, -COOR¹⁵ and CONR¹⁶R¹⁷, wherein
R¹⁵ and R¹⁶ are independently selected from hydrogen and methyl, and
R¹⁷ is selected from hydrogen, methyl, 4-hydroxybutyl and 2-tetrahydrofuryl;
R⁹ is selected from phenyl which is optionally substituted, and hetaryl which is optionally substituted;
R¹⁰ is selected from hydrogen, halogen, alkyl and alkoxy, and
R¹¹ is selected from hydrogen, hydroxyl, halogen, alkoxy and alkyl; or
R¹⁰ and R¹¹ are in ortho-position to each other and together form a -OCH₂O- unit;
or a pharmaceutically acceptable salt thereof, for the manufacture of a medicament for the treatment of inflammatory diseases,
wherein the rocaglamide derivative of the formula (I) is used in a therapeutically effective amount which enhances the phosphorylation of NF-AT in leukocytes of a patient but which does essentially not inhibit NF-kappa B activity and AP-1 activity.

2. The use according to claim 1, wherein the therapeutically effective amount inhibits the production of cytokines.

3. The use according to claim 2, wherein the cytokines are selected from the group consisting of IFN-γ, TNF-α, GM-CSF, IL-1, IL-2, IL-4, IL-5, IL-6, IL-8, and IL-12.

4. The use according to any of claims 1 to 3, wherein the leukocytes are selected from the group consisting of T helper cells, T killer cells, macrophages, granulocytes, and monocytes.

5. The use according to any of claims 1 to 4, wherein the therapeutically effective amount comprises a blood plasma concentration in a range of from 15 nM to 100 nM, preferably of from 18 nM to 90 nM, more preferably of from 20 nM to 70 nM, most preferably of from 23 nM to 50 nM, and even most preferably of from 25 nM to 30 nM

6. The use according to any of claims 1 to 5, wherein the phosphorylation of NF-AT is enhanced at least 1.5-fold, at least 2-fold, at least 4-fold, at least 8-fold, at least 10-fold, at least 20-fold, at least 50-fold, at least 100-fold.

7. The use according to any of claims 1 to 6, wherein the inflammatory disease is a chronic inflammatory disease, an acute inflammatory disease, an allergic inflammatory disorder, a graft-versus-host rejection disease, and/or an autoimmune disorder.

8. The use according to any of claims 1 to 7, wherein the inflammatory disease is a disease of the airways and lung, a skin disease, rheumatoid arthritis, rhinoconjunctivities, anaphylaxis, and/or cardiac diseases, preferably a disease of the airways and lung.

9. A method for preparing a medicament which contains a rocaglamide derivative of formula (I) and, optionally, a pharmaceutically acceptable carrier, the method comprising admixing in a suitable amount the rocaglamide derivative optionally with a pharmaceutically acceptable carrier, preservative and/or buffer, wherein the rocaglamide derivative of the formula (I) is used in a therapeutically effective amount which enhances the phosphorylation of NF-AT in leukocytes of a patient but which does essentially not inhibit NF-kappa B activity and AP-1 activity.

10. A medicament for the treatment of inflammatory diseases which comprises a rocaglamide derivative of formula (I) and, optionally, a pharmaceutically acceptable carrier,
wherein the rocaglamide derivative of the formula (I) is used in a therapeutically effective amount which enhances the phosphorylation of NF-AT in leukocytes of a patient but which does essentially not inhibit NF-kappa B activity and AP-1 activity.

11. The medicament according to claim 10, wherein the therapeutically effective amount comprises a blood plasma concentration in a range of from 15 nM to 100 nM, preferably of from 18 nM to 90 nM, more preferably of from 20 nM to 70 nM, most preferably of from 23 nM to 50 nM, and even most preferably of from 25 nM to 30 nM.

12. The medicament according to claim 10 or 11, wherein the inflammatory disease is a disease as defined in claims 7 and 8.

13. A method of inhibiting cytokine production in cells, the method comprising the step of treating said cells with a rocaglamide derivative of the formula (I), wherein the rocaglamide derivative of the formula (I) is used in a therapeutically effective amount which enhances the phosphorylation of NF-AT in leukocytes but which does essentially not inhibit NF-kappa B activity and AP-1 activity

14. The method according to claim 13, wherein the cytokine is selected from the group consisting of IFN-γ, TNF-α, GM-CSF, IL-1, IL-2, IL-4, IL-5, IL-6, IL-8, and IL-12.

15. The method according to claim 14, wherein the cytokine is IFN-γ, TNF-α, IL-2 and/or IL-4.

16. The method according to any of claims 13 to 15, wherein the effective amount of the rocaglamide derivative comprises a concentration in a range of from 15 nM to 100 nM, preferably of from 18 nM to 90 nM, more preferably of from 20 nM to 70 nM, most preferably of from 23 nM to 50 nM, and even most preferably of from 25 nM to 30 nM.


SEQUENCE LISTING

<110> Deutsches Krebsforschungszentrum (German Cancer Research Center)

<120> Use of rocaglamide derivatives as NF-AT-specific inhibitors for the
treatment of certain inflammatory diseases

<130> DK62843EP

<160> 6

<170> PatentIn version 3.1

<210> 1
<211> 20
<212> DNA
<213> artificial sequence

<220>
<223> IFN-alpha forward primer

<400> 1
ggagaagggt gaccgactca 20

<210> 2
<211> 17
<212> DNA
<213> artificial sequence

<220>
<223> IFN-alpha reverse primer

<400> 2
tgcccagact cggcaag 17

<210> 3
<211> 24
<212> DNA
<213> artificial sequence

<220>
<223> IFN-alpha probe

<400> 3
cgctgagatc aatcggcccg acta 24

<210> 4
<211> 17
<212> DNA
<213> artificial sequence

<220>
<223> SV 40 enhancer

<400> 4
cggttgctga ctaattg 17

<210> 5
<211> 11

<212>  DNA
<213>  artificial sequence

<220>
<223>  NF-kappa B consensus sequence

<400>  5
ggaaattccc c                                                          11


<210>  6
<211>  32
<212>  DNA
<213>  artificial sequence

<220>
<223>  human IL-2 NF-AT element

<400>  6
gaaaggagga aaaactgttt catacagaag gc                                   32